(19) Europäisches Patentamt
European Patent Office
Office européen des brevets

(11) Veröffentlichungsnummer : **0 163 107**
**B1**

(12) # EUROPÄISCHE PATENTSCHRIFT

(45) Veröffentlichungstag der Patentschrift : **30.09.87**

(51) Int. Cl.⁴ : **C 07 C101/40**, C 07 D295/14, C 23 F 11/14

(21) Anmeldenummer : **85104839.7**

(22) Anmeldetag : **22.04.85**

(54) **Verwendung von Benzoylalaninen als Korrosionsinhibitoren für wässrige Systeme.**

(30) Priorität : **30.04.84 DE 3416120**

(43) Veröffentlichungstag der Anmeldung : **04.12.85 Patentblatt 85/49**

(45) Bekanntmachung des Hinweises auf die Patenterteilung : **30.09.87 Patentblatt 87/40**

(84) Benannte Vertragsstaaten : **AT BE CH DE FR GB IT LI NL SE**

(56) Entgegenhaltungen :
**CHEMICAL ABSTRACTS, Band 82, Nr. 7, 17 Februar 1975, Seite 469, Nr. 43710s, Columbus, Ohio, US; S. CERIANI et al.: "*Simple synthesis of DL-bêta-amino-bêta-aroylpropionic acids*"**
**CHEMICAL ABSTRACTS, Band 80, Nr. 19, 13 Mai 1974, Seite 416, Nr. 108473x, Columbus, Ohio, US; A. SAMMOUR et al.: "Reactions with B-aroylacryic acids"**
**CHEMICAL ABSTRACTS, Band 91, Nr. 25, 17 Dezember 1979, Seite 676, Nr. 211312v, Columbus, Ohio, US; A. SAMMOUR et al.: "Behavior of bêta-aroylacrylic acids towards the action of amines, thioureas, aromatic hydrocarbons, thiophenol and active methylene compounds"**
**BULLETIN DE LA SOCIETE CHIMIQUE DE FRANCE, Nr. 9, 1971, Seiten 3196-3199, Paris, FR; J. COUQUELET et al.: "Amino-alcoylations avec l'acide glyoxylique: application à quelques cétones aromatiques et hétérocycliques"**
**CHEMICAL ABSTRACTS, Band 67, 1967, Seite 6003, Nr. 63985d, Columbus, Ohio, US; K. HIROTSUGU et al.: "Addition of bêta-benzoylacrylic acid and its ethyl ester to ethanolamine"**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Geke, Jürgen, Dr.**
**Stoffeler Damm 108**
**D-4000 Düsseldorf 1 (DE)**
Erfinder : **Penninger, Josef, Dr.**
**Mozartstrasse 64**
**D-4010 Hilden (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 163 107 B1**

Jouve, 18, rue St-Denis, 75001 Paris, France

CHEMICAL ABSTRACTS, Band 89, Nr. 15, 9 Oktober 1978, Seite 560, Nr. 129191e, Columbus, Ohio, US; S.G. AGBALYAN et al.: "Reaction of bêta-aroylacrylic acids with monoethanolamine and diethanolamine"
CHEMICAL ABSTRACTS, Band 89, Nr. 25, 18 Dezember 1978, Seite 630, Nr. 215732p, Columbus, Ohio, US; S.G. AGBALYAN et al.: "Addition of aliphatic amino acids to bêta-aroylacrylic acids"
Chemical Abstracts, vol. 97 (1982), p. 566, abstract 161229z
Chemical Abstracts, vol. 71 (1969), p. 439, abstract 91863p

**Beschreibung**

Die Erfindung betrifft die Verwendung spezieller Benzoylalanine als Korrosionsinhibitoren für wäßrige Systeme.

In zahlreichen technischen Prozessen, in denen wäßrige Medien mit metallischen Oberflächen, vornehmlich mit Oberflächen aus Eisen, Kupfer, Aluminium, Zink oder deren vielseitigen Legierungen in Berührung kommen, treten Korrosionsprobleme auf. Beispiele dafür sind Reinigungsprozesse mit wäßrigen technischen Reiniger-Lösung, Kühlr-Prozesse mit wasserhaltigen Kühlmittel-Systemen sowie die Kühlung und gleichzeitige Schmierung in der Bearbeitung von Metallen.

So sind in der DE-B-1 149 843 Halbamide der Maleinsäure oder Bernsteinsäure als Zusatzmittel für Brennstoff- und Schmieröle offenbart. Ein Nachteil dieser Verbindungen ist es jedoch, daß sie in den meisten Fällen nicht wasserlöslich sind und damit eine homogene Verteilung in der gesamten Prozeßflüssigkeit nicht gewährleistet werden kann.

Die EP-B-0 002 780 offenbart ebenfalls Halbamide der Maleinsäure als Korrosionsinhibitoren für wässrige Systeme, wobei für die am Amid-Stickstoff gebundenen Alkylgruppen ein C-Zahlbereich von 9 bis 12 angegeben wird und außerdem die entstehenden Amidsäuren mit Mono-, Di- oder Trialkanolaminen oder deren Gemischen neutralisiert werden.

Als Korrosionsinhibitoren für Eisen im alkalischen Medium sind außerdem Alkenylbernsteinsäuren (DE-A-29 43 963), langkettige Sulfonamidocarbonsäuren (DE-B-1 298 670), Acylsarkosinate (Winnacker-Küchler, Chemische Technologie, C. Hanser-Verlag, München (1960), Seite 199) oder Alkalimetall-Benzoate vorgeschlagen worden. Für Eisen in schwach sauren Medien wurden bisher meist Fettamine oder Imidazoline verwendet, ohne daß sich damit ein vollauf befriedigender Erfolg erzielen ließe.

Als Korrosionsinhibitoren für Aluminium im alkalischen Medium finden meist Alkalimetall-Silikate oder Alkalimetall-Benzoate Verwendung, wobei auch diese den Anforderungen oft nicht gerecht werden. Besonders nachteilig wirkt sich bei den erwähnten Verbindungen aus, daß sie nur in sehr hohen Konzentrationen wirksam sind.

Die Verwendung von Korrosionsinhibitoren der genannten Art führt dabei häufig neben ungenügendem Korrosionsschutz zu einer Reihe anwendungstechnischer Schwierigkeiten. Starkes Schäumen der Verbindungen in wässrigen Lösungen, schlechte Wasserlöslichkeit und/oder schlechte Wasserhärtestabilität oder unzureichende Lagerstabilität führen zu einer erheblichen Einschränkung in der praktischen Verwendung einiger der genannten Verbindungen. Dabei muß auch ein Augenmerk auf die oft zu hohe Toxizität der Verbindungen sowie ihre äußerst schlechte biologische Abbaubarkeit gerichtet werden.

Spezielle Benzoylalanine sind aus der nachstehend angeführten Literatur bereits bekannt :

Chemical Abstracts, Vol. 80 (1974), Seite 416, Abstract 108 473x : Herstellung von Verbindungen des Typs $R_1COCH_2CHRCO_2H$, mit R = Piperidino, Morpholino, BuNH ; $R_1 = MeC_6H_4$.

Chemical Abstracts, Vol. 91 (1979), Seite 676, Abstract 211 312v : Herstellung von Verbindungen des Typs $RCOCH_2CHR_1CO_2H$, mit R = 2,4- und 2,5-Xylyl ; $R_1$ = Piperidino, Morpholino.

Bulletin de la Société Chimique de France (1971), Seiten 3196 bis 3199 : Hier werden auf Seite 3197, unter Nr. 1 und 7, Verbindungen des Typs $R\text{-}C_6H_4\text{-}COCH_2CH(R_1)CO_2H$, mit R = H oder $CH_3$ und $R_1$ = Morpholinrest, sowie deren Herstellung beschrieben.

Chemical Abstracts, Vol. 82 (1975), Seite 469, Abstract 43 710s : Herstellung von Verbindungen des Typs $R\text{-}C_6H_4\text{-}COCH_2CH(NH_2)\ CO_2H$, mit R = H oder Me.

Chemical Abstracts, Vol. 67 (1967), Seite 6003, Abstract 63 985d : Herstellung von Verbindungen des Typs $BzCH_2CH(NH_2)\ CO_2H$, mit Bz = Benzoyl.

Chemical Abstracts, Vol. 89 (1978), Seite 560, Abstract 129 191e : Herstellung von Verbindungen des Typs $RCOCH_2CH(CO_2H)\ NHCH_2CH_2OH$ und $PhCOCH_2CH\ [N(CH_2CH_2OH)_2]\ CO_2^-$, mit R = Phenyl (Ph), p-Tolyl.

Chemical Abstracts, Vol. 89 (1978), Seite 630, Abstract 215 732p : Herstelung von Verbindungen des Typs $RCOCH_2CHR_1CO_2H$, mit R = Phenyl, $4\text{-}MeC_6H_4$ ; $R_1$ = Glycin-OH.

Chemical Abstracts, Vol. 97 (1982), Seite 566, Abstract 161 229z : Vergleich der pharmakologischen Eigenschaften von α-(2-Hydroxyethylamino)-β-benzoylpropionsäure und Natrium-Salicylat.

Chemical Abstracts, Vol. 71 (1969), Seite 439, Abstract 91 863p : Hydrolyse von Kynurenin-Peptiden. Unter anderen wird hier die Synthese von α-Amino-β-benzoylpropionsäure beschrieben.

Es wurde nun gefunden, daß man wäßrige Systeme mit ausgezeichneten Korrosionsschutz-Eigenschaften, hoher Wasserlöslichkeit und geringem Schaumvermögen erhält, wenn man als Korrosionsinhibitoren spezielle Benzoylalanine verwendet.

Die Erfindung betrifft somit die Verwendung von Verbindungen der allgemeinen Formel I

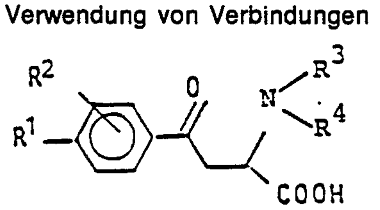

(I)

in der

$R^1$ und $R^2$ Wasserstoff oder einen Alkylrest mit 1 bis 12 C-Atomen bedeuten und

$R^3$ und $R^4$ gleich oder verschieden sein können und für Wasserstoff, einen unverzweigten oder verzweigten Alkylrest mit 1 bis 10 C-Atomen, Hydroxyalkylen-, Alkoxyalkylen-, Carboxyalkylen- oder Alkylaminoalkylen-Reste stehen, die 1 bis 3 C-Atomen, Hydroxyalkylen-, Alkoxyalkylen-, Carboxyalkylen- oder Alkylaminoalkylen-Reste stehen, die 1 bis 3 C-Atome im Alkylenrest und 1 bis 3 C-Atomen im Alkylrest enthalten und auch zu einem gesättigten heterocyclischen 5- oder 6-Ring geschlossen sein können,
oder deren Alkali- bzw. Ammoniumsalze mit Ammoniak, Mono-, Di- und Triethanolamin, in Mengen von 0,001 bis 0,375 Gew.-% als Korrosionsinhibitoren in wäßrigen Systemen.

Insbesondere sind als Korrosionsinhibitoren solche Verbindungen der allgemeinen Formel I geeignet, in denen $R^1$ ein Alkylrest mit 1 bis 8 C-Atomen, bevorzugt Methyl, Ethyl, Propyl, i-Propyl, n-Butyl, tert.-Butyl oder n-Hexyl, $R^2$ Wasserstoff oder ein Methylrest und entweder $R^3$ Wasserstoff und $R^4$ Wasserstoff, ein Alkylrest mit 1-4 C-Atomen, bevorzugt Ethyl oder n-Butyl-, ein Hydroxyethylen-, Carboxymethylen- oder Dimethylaminopropyl-Rest oder $R^3$ und $R^4$ je ein Hydroxyethylenrest oder $R^3$ und $R^4$ gemeinsam für einen Ethoxyethylen- oder Ethyliminoethylenrest stehen, d. h. unter Einschluß des sie bindenden Stickstoffatoms zu einem Morpholino- oder Piperidino-Rest verbunden sind.

Die Benzoylalanine können gemäß der Erfindung als Korrosionsschutzmittel einzeln oder in Mischungen miteinander in beliebigem Verhältnis verwendet werden. Sie entfalten ihre vorteilhaften Wirkungen in wäßrigen Lösungen, Dispersionen oder Emulsionen. Bereits bei geringen Konzentrationen ist eine hohe Wirksamkeit vorhanden. So genügen beispielsweise für den Korrosionsschutz von Eisenoberflächen im alkalischen Medium bereits Konzentrationen von 0,5 $kg \cdot m^{-3}$ um eine hohe Wirksamkeit des Korrosionsschutzmittels zu erzielen, während bisher übliche Inhibitoren in Konzentrationen von 2,5 bis 10 $kg \cdot m^{-3}$ angewendet werden müssen. Für Eisenoberflächen im Kontakt mit schwach sauren wäßrigen Medien sind überraschenderweise sogar Konzentrationen um 0,1 $kg \cdot m^{-3}$ für einen guten Schutz ausreichend. Dies ist umso überraschender, da in der Literatur mit den oben erwähnten Imidazolinen und Fettaminen bisher nur ganz wenige Verbindungen beschrieben sind, die in diesem pH-Bereich vor Korrosion schützen. Dieser Schutz ist jedoch gering, wie aus den vergleichenden Versuchen hervorgeht, deren Ergebnisse den Tabellen 1 bis 3 zu entnehmen sind.

Weiterhin wurde gefunden, daß für Aluminiumoberflächen im Kontakt mit alkalischen Medien bereits Konzentrationen der genannten Benzoylalanine von 0,01 $kg \cdot m^{-3}$ für einen optimalen Schutz sorgen.

Die erfindungsgemäß verwendeten Benzoylalanine weisen in den zur Anwendung kommenden Konzentrationen eine ausgeprägte Schaumarmut auf und besitzen in allen verwendeten Wässern eine gute Wasserhärte-Stabilität. Dies ermöglicht ihre Verwendung in wäßrigen Systemen jeder Zusammensetzung, z. B. in Reinigungsmitteln auf wäßriger Basis, Schmiermitteln, Kühlkreisläufe, Hydraulikflüssigkeiten usw.

Die Herstellung der erfindungsgemäß verwendbaren Benzoylalanine erfolgt nach an sich bekannten Methoden. Sie können beispielsweise vorteilhaft durch Friedel-Crafts-Acylierung von Alkylbenzolen mit Maleinsäureanhydrid und anschließender Addition von Aminen an die Doppelbindung der im ersten Reaktionsschritt erhaltenen 3-Benzoyl-acrylsäuren in guten Ausbeuten synthetisiert werden. Den aufgezeigten Darstellungsweg zeigt das nachfolgende Reaktionsschema.

Reaktionsschema

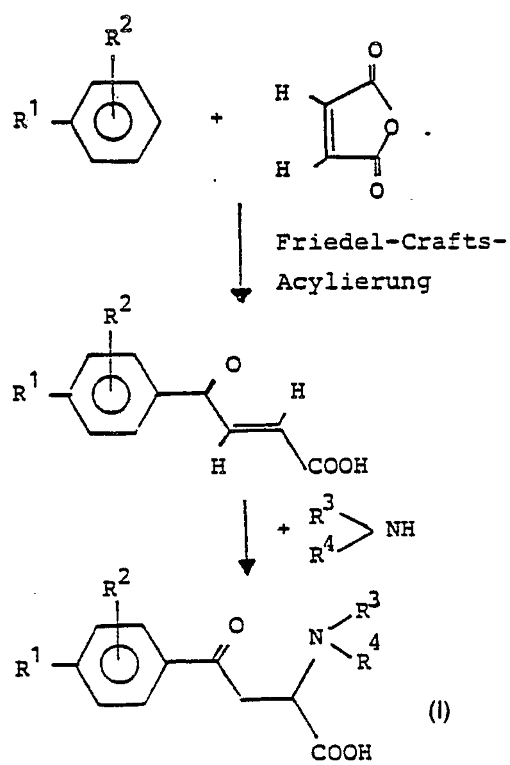


(I)

Für die jeweiligen die Benzoylalanine als Korrosionsinhibitoren enthaltenden wäßrigen Systeme werden die Verbindungen der Formel I oder deren Alkali- bzw. Ammoniumsalze nach an sich bekannten Methoden unmittelbar gelöst oder in Form wäßriger Konzentrate den jeweiligen wäßrigen Systemen zugemischt.

Der Anmeldungsgegenstand wird durch die nachfolgenden Beispiele näher erläutert.

Die Bestimmung der Korrosionsschutzeigenschaften erfolgte durch Ermittlung des Massenabtrags (DIN 50905/1-4) sowie des Filterpapiertests (DIN 51360/2).

## Beispiel 1

Massenabtragstest :

Je drei sorgfältig vorbehandelte und gewogene Metallstreifen (unlegierter Stahl, 80 × 15 × 1 mm) wurden in ein 1-1-Gefäß, das 800 ml Testwasser, 50 ml Pufferlösung sowie eine definierte Menge an je einem Benzoylalanin I gemäß der Erfindung enthielt, gehängt und 3 h bei Raumtemperatur darin belassen. Die Lösung wurde mit einer Geschwindigkeit von 80 U. $min^{-1}$ gerührt.

Das als korrosives Medium benutzte Versuchswasser wurde nach DIN 51360/2 hergestellt und mit Ammoniak/Ammoniumchlorid auf einen pH-Wert von 9,0 gepuffert.

Nach Ablauf der Versuchszeit wurden die Metallstreifen getrocknet und gewogen. Aus dem Gewichtsverlust wurde der Korrosionsschutzwert S, bezogen auf eine Blindprobe, berechnet :

$$S = 100\,(1 - a/b)$$

a = Gewichtsverlust der Probe
b = Gewichtsverlust der Blindprobe

Die Ergebnisse des Massenabtragstests sind in Tabelle 1 wiedergegeben.

Tabelle 1

| $R_1$ | $R_2$ | $R_3$ | $R_4$ | Base | Korrosionsschutzwert S 0,25[a] | 0,1[a] | 0,05[a] |
|---|---|---|---|---|---|---|---|
| $CH_3$ | $CH_3$ | H | $CH_2CH_2OH$ | $NH_3$ | 95 | 94 | 30 |
| $(CH_3)_2CH$ | H | H | " | " | 99 | 95 | 95 |
| " | " | " | " | DEA[b] | 96 | 90 | 42 |
| $(CH_3)_3C$ | " | " | " | $NH_3$ | 99 | 99 | 90 |
| " | " | " | " | MEA[c] | 97 | 96 | 66 |
| $n\text{-}C_6H_{13}$ | " | " | $(CH_2)_3N(CH_3)_2$ | $NH_3$ | 92 | 91 | 85 |
| $CH_3$ | " | " | $(CH_2)_3CH_3$ | " | 94 | 64 | 40 |
| $CH_2CH_3$ | " | " | " | " | 99 | 98 | 89 |
| $CH_3$ | $CH_3$ | " | " | " | 91 | 81 | 30 |
| Na-benzoat (Vergleich) | | | | | 52 | 18 | 0 |
| Maleinsäuremono-2-ethylhexyl-amid (Vergleich) | | | | | 69 | 0 | 0 |
| Benzolsulfonamidocapronsäure (Vergleich) | | | | | 65 | 0 | 0 |

Erläuterungen :
a) Inhibitorenkonzentation in Gew.-%
b) DEA = Diethanolamin
c) MEA = Monoethanolamin

## Beispiel 2

Grauguß-Filterpapiertest :

Die Durchführung des Grauguß-Filterpapiertest erfolgte gemäß DIN 51360/2. Als Testmedium wurde Wasser mit einer Härte von 20 °d entsprechend der DIN-Anweisung verwendet. Die beanspruchten

Verbindungen wurden in Form des Diethanolamin-Salzes (pH 9,7) geprüft.

Die Bewertung wurde entsprechend obiger DIN-Norm in Korrosionsgrade vorgenommen :

0 = keine Korrosion
1 = Spuren von Korrosion
2 = leichte Korrosion
3 = mäßige Korrosion
4 = starke Korrosion

Die Prüfergebnisse sind in Tabelle 2 aufgeführt.

Tabelle 2

| $R_1$ | $R_2$ | $R_3$ | $R_4$ | Korrosionsschutzgrad 0,375[a] | 0,250[a] | 0,125[a] |
|---|---|---|---|---|---|---|
| Me | Me | H | $CH_2CH_2OH$ | 0 | 0 | 1 |
| " | H | " | " | 0 | 1 | 2 |
| $Me_2CH$ | " | " | " | 0 | 0 | 2 |
| $Me_3C$ | " | " | $(CH_2)_3N(CH_3)_2$ | 0 | 1 | 2 |
| $n-C_6H_{13}$ | " | " | " | 0 | 0 | 2 |
| Me | Me | " | $(CH_2)_3CH_3$ | 0 | 0 | 2 |
| Me | H | " | " | 0 | 0 | 2 |
| Et | " | " | " | 0 | 0 | 1 |
| $n-C_4H_9$ | " | $CH_2CH_2OH$ | $CH_2CH_2OH$ | 0 | 0 | 1 |
| $CH_3$ | " | H | $CH_2CH_3$ | 0 | 0 | 3 |
| $(CH_3)_2CH$ | " | " | $CH_2COOH$ | 0 | 1 | 3 |
| " | " | Ethoxyethylen | | 0 | 1 | 2 |
| Vergleich: | | | | | | |
| Caprylsäure | | | | 3 | 3 | 4 |
| Maleinsäure-mono-2-ethylhexylamid | | | | 0 | 1 | 3 |
| Benzolsulfonamidocapronsäure | | | | 1 | 1 | 3 |

Erläuterungen :
[a]) Inhibitorkonzentration in Gew.-%.

## Beispiel 3

Durchführung analog Beispiel 1.
Pufferung des Mediums mit HOAc/NaOAc auf pH 4.
Die Korrosionsschutzwerte sind in Tabelle 3 wiedergegeben.

Tabelle 3

| $R_1$ | $R_2$ | $R_3$ | $R_4$ | Korrosionsschutzwert S 0,05[a] | 0,025[a] | 0,01[a] |
|---|---|---|---|---|---|---|
| $(CH_3)_2CH$ | H | H | $(CH_2)_3N(CH_3)_2$ | 91 | 81 | 65 |
| $(CH_3)_3C$ | " | " | " | 92 | 91 | 87 |
| Vergleich 1[b] | | | | 34 | 28 | 27 |
| Vergleich 2[c] | | | | 33 | 29 | 28 |

Erläuterungen :
[a]) Inhibitorkonzentration in Gew.-%
[b]) Vergleich 1 = 1-(2'-Hydroxyethyl)-2-oleyl-imidazolin
[c]) Vergleich 2 = 1-(2'-Aminoethyl)-2-oleyl-imidazolin

Beispiel 4

Durchführung analog Beispiel 1.
Metallstreifen : Aluminium, 80 × 15 × 1 mm
Testzeit : 65 h
Die beanspruchten Verbindungen wurden in Form der Na-Salze geprüft.
Die Korrosionsschutzwerte sind in Tabelle 4 aufgeführt.

Tabelle 4

| $R_1$ | $R_2$ | $R_3$ | $R_4$ | Korrosionsschutzwert S $0,05^{a)}$ | $0,025^{a)}$ | $0,01^{a)}$ |
|---|---|---|---|---|---|---|
| $(CH_3)_2CH$ | H | H | $CH_2CH_2OH$ | 96 | 28 | 22 |
| $(CH_3)_3C$ | " | " | " | 99 | 45 | 32 |
| $n-C_6H_{13}$ | " | " | " | 99 | 99 | 99 |
| " | " | " | $(CH_2)_3N(CH_3)_2$ | 99 | 99 | 28 |

Erläuterungen :
a) Inhibitorkonzentration in Gew.-%.

Vergleich : Na-Benzoat ergibt bei diesen Konzentrationen keinerlei Korrosionsschutz mehr.

Wenn vor- und nachstehend von Alkylen-Resten die Rede ist, so sind damit Kohlenwasserstoffreste gemeint, die je eine freie Valenz an den endständigen C-Atomen der C-Kette aufweisen. Im Sinne einer korrekten Nomenklatur sind diese Reste als Alkylreste zu bezeichnen ; anstelle von Hydroxyalkylen-, Alkoxyalkylen- oder Carboxyalkylen-Resten muß es korrekterweise heißen : Hydroxyalkyl-, Alkoxyalkyl- oder Carboxyalkyl-Reste.

**Patentansprüche**

1. Verwendung von Benzoylalaninen der allgemeinen Formel I

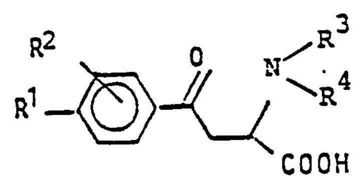

(I)

in der

$R^1$ und $R^2$ Wasserstoff oder einen Alkylrest mit 1 bis 12 C-Atomen bedeuten und

$R^3$ und $R^4$ gleich oder verschieden sein können und für Wasserstoff, einen unverzweigten oder verzweigten Alkylrest mit 1 bis 10 C-Atomen, Hydroxyalkylen-, Alkoxyalkylen-, Carboxyalkylen- oder Alkylaminoalkylen-Reste stehen, die 1 bis 3 C-Atome im Alkylenrest und 1 bis 3 C-Atome im Alkylrest enthalten und auch zu einem gesättigten heterocyclischen 5- oder 6-Ring geschlossen sein können, oder deren Alkali- bzw. Ammoniumsalze mit Ammoniak, Mono-, Di- und Triethanolamin in Mengen von 0,001 bis 0,375 Gew.-% als Korrosionsinhibitoren in wässrigen Systemen.

2. Verwendung von Benzoylalaninen nach Anspruch 1, dadurch gekennzeichnet, daß in der allgemeinen Formel I

$R^1$ ein Alkylrest mit 1 bis 8 C-Atomen,

$R^2$ Wasserstoff oder ein Methylrest und

$R^3$ und $R^4$ gleich oder verschieden sind und für Wasserstoff, einen unverzweigten oder verzweigten Alkylrest mit 1 bis 10 C-Atomen, einen oder zwei Hydroxyethylen-Reste, einen Carboxymethylen-Rest, einen Dimethylaminopropylen-Rest oder gemeinsam für einen Ethoxyethylen-Rest stehen.

**Claims**

1. The use of benzoyl alanines corresponding to the following general formula

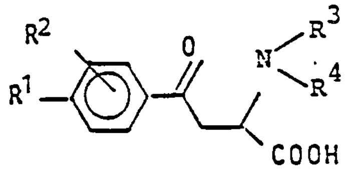

(I)

in which

$R^1$ and $R^2$ represent hydrogen or a $C_1$-$C_{12}$ alkyl radical and

$R^3$ and $R^4$, which may be the same or different, represent hydrogen, an unbranched or branched $C_1$-$C_{10}$ alkyl radical, hydroxyalkylene, alkoxyalkylene, carboxyalkylene or alkylaminoalkylene radicals which contain from 1 to 3 C-Atoms in the alkylene radical and from 1 to 3 C-atoms in the alkyl radical and may also be closed to form a saturated heterocyclic 5- or 6-membered ring, or alkali or ammonium salts thereof with ammonia, mono-, di- and triethanolamine in quantities of from 0.001 to 0.375 % by weight as corrosion inhibitors in aqueous systems.

2. The use of benzoyl alanines as claimed in Claim 1, characterized in that, in general formula I,

$R^1$ is a $C_1$-$C_8$ alkyl radical,

$R^2$ is hydrogen or a methyl radical and

$R^3$ and $R^4$, which may be the same or different, represent hydrogen, an unbranched or branched $C_1$-$C_{10}$ alkyl radical, one or two hydroxyethylene radicals, a carboxymethylene radical, a dimethylamino propylene radical or together may represent an ethoxyethylene radical.

**Revendications**

1. Utilisation de benzoylalanines de formule générale I :

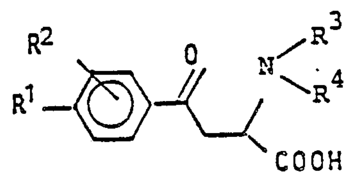

(I)

dans laquelle

$R^1$ et $R^2$ représentent de l'hydrogène ou un radical alcoyle ayant 1 à 12 atomes de carbone et

$R^3$ et $R^4$ peuvent être identiques ou différents et représentent de l'hydrogène, un radical alcoyle à chaîne droite ou ramifiée ayant 1 à 10 atomes de carbone, des radicaux hydroxyalcoylène, alcoxyalcoylène, carboxyalcoylène ou alcoylaminoalcoylène qui contiennent 1 à 3 atomes de carbone dans le radical alcoylène et 1 à 3 atomes de carbone dans le radical alcoyle et qui peuvent aussi se cycliser en un noyau à 5 ou 6 chaînons saturé hétérocyclique, ou de leurs sels alcalins ou d'ammonium avec l'ammoniac, la mono-, di- et triéthanolamine, en des quantités de 0,001 à 0,375 % en poids, en tant qu'inhibiteurs de corrosion dans des systèmes aqueux.

2. Utilisation de benzoylalanines selon la revendication 1, caractérisée en ce que dans la formule générale I

$R^1$ est un radical alcoyle ayant 1 à 8 atomes de carbone,

$R^2$ de l'hydrogène ou un radical méthyle et

$R^3$ et $R^4$ sont identiques ou différents et peuvent représenter de l'hydrogène, un radical alcoyle à chaîne droite ou ramifiée ayant 1 à 10 atomes de carbone, un ou deux radicaux hydroxyéthylène, un radical carboxyméthylène, un radical diméthylaminopropylène ou ensemble un radical éthoxyéthylène.